# EUROPEAN PATENT APPLICATION

(11) **EP 4 282 418 A1**
(43) Date of publication of application: **29.11.2023**
(21) Application number: 22758650.0
(22) Date of filing: 27.01.2022
(51) Int. Cl.: A61K 31/506, A61K 31/19, A61Q 7/00, A61P 17/14

(54) **FOAMING TOPICAL PHARMACEUTICAL COMPOSITION**

(30) Priority: 24.02.2021 BR 102021003531
(71) Applicant: Eurofarma Laboratórios S.A., 04603-003 São Paulo - SP (BR)
(72) Inventor: DE OLIVEIRA FARIA, Luiz Felipe, 05468-140 São Paulo (BR); SANTOS COSTA, Maiara, 06172-280 São Paulo (BR)
(74) Representative: Michalski Hüttermann & Partner Patentanwälte mbB
(86) International application number: PCT/BR2022/050021
(87) International publication number: WO 2022/178604

(57) **Abstract**

**TOPICAL FOAMABLE PHARMACEUTICAL COMPOSITION,** which refers to a topical pharmaceutical composition, having a pleasant feeling that inhibits the formation of active crystals, and its use for the treatment of alopecia and other related disorders.

## Description

### Field of the Invention

The present invention refers to a topical foamable pharmaceutical composition, with a pleasant feeling, which inhibits the crystal formation of the active Minoxidil. The present invention lies in the fields of Pharmacotechnics and pharmaceutical technology.

### Background of the Invention

Androgenic alopecia or male baldness is the most common type of hair loss in men, affecting up to 70% of all men in their adult lives. Androgenic alopecia occurs in the hair follicles in a certain area of the scalp, leading to follicular miniaturization, process by which the hair shaft becomes thinner and shorter. Although in this process the follicles remain alive, there is no new hair growth.

Women can also develop androgenic alopecia. The mechanism of action of the androgenic component is not fully understood and the clinical presentation is different. Therefore, currently the use of the term Female Pattern Alopecia (FPA) is preferred instead of female androgenic alopecia.

Minoxidil is a potent vasodilator and is currently indicated for the treatment of androgenic alopecia and female pattern alopecia. Its mechanism of action as a hair growth stimulator has not yet been fully elucidated but it is believed that the drug acts on different pathways culminating in stimulating hair growth, such as:
- Vasodilation of the microcirculation around the hair follicles stimulating hair growth;
- Stimulation of vascular endothelial growth factor (VEGF);
- Stimulation for the follicles to pass to the growth phase (anagenesis);
- Prolongation of the growth phase (anagenesis).

Other studies suggest Minoxidil works by opening adenosine triphosphate (ATP)-sensitive potassium channels in vascular smooth muscle, causing it to relax and reducing blood pressure in hair follicles.

In this way, Minoxidil's action is restricted to the hair follicles and the scope of the treatment is to reach the hair bulb in effective concentrations and at the same time prevent its passage to the deeper layers of the skin and, subsequently, to the bloodstream, obtaining only a topical local effect. In this case, the measure of systemic absorption of the drug is more related to safety than to local efficacy, which for Minoxidil must be below the concentration associated with adverse effects (20 ng/mL).

This balance between good skin permeation to improve therapeutic efficiency and low systemic exposure to minimize side effects depends on factors related to drug solubility and formulation.

The prior art shows that Minoxidil has low water solubility (2.2 mg/mL), moderate ethanol solubility (29 mg/mL) and high propylene glycol solubility (75 mg/mL). Thus, Minoxidil solubility is the physical-chemical parameter comprising the greatest challenge to be overcome in terms of formulation.

Only a solution containing 75% propylene glycol would allow the drug to be completely soluble in the medium, considering the application of a dose of 50 mg/mL. These data explain the fact that preparations containing Minoxidil have low water content and high concentrations of ethanol and/or propylene glycol.

Currently, Minoxidil can be found in different pharmaceutical forms, such as immediate release tablet (Loniten^{®}), topical capillary solution (Rogaine^{®} solution, Aloxidil^{®}, Pant^{®}, among others) and foam available only in some countries under the trade name of Rogaine^{®} Foam and generic products with the same qualitative composition.

The solution pharmaceutical form was registered for the first time in the United States in 1996. In the prior art there are several documents describing the formulation of Minoxidil in solution such as WO8801502, WO9927966, WO0176541, WO0211698, WO02092092 and WO2012106249.

Commercial solution products usually have a slightly yellowish solution and an oily texture, including ethanol, propylene glycol and water in its composition. The primary packaging is usually a vial with a spray valve for application or a dropper pipette.

The foam pharmaceutical form was registered for the first time in 2006, in the United States, by the company Pharmacia & Upjohn (from the Pfizer group) under the trade name Rogaine^{®}. The drug composition is described in US6946120. This reports a topical composition with at least 5% (w/w) of Minoxidil, containing water and/or short-chain acyl alcohol, a co-solvent, and pH less than or equal to 7. The patent specification presents 12 different examples of formulation with varied proportions and alternative types of acid and co-solvents. It is notable that in all examples the percentage of ethanol is close to or exceeds 50% formulation weight; water-insoluble surfactants are also used, giving rise to an emulsion, which in this case makes it possible to apply the product through only a package containing a propelling agent, as stated in its set of claims.

The high pressure due to the propelling agent enables the expansion of the liquid at high rate and the formation of a large number of small bubbles. Although the bubbles have tendency to agglomerate and coalesce, the high viscosity slows the liquid flow and the waxy surfactants form an "insoluble" adsorption layer around the bubbles.

US4828837 and US2005079139 also disclose Minoxidil foam formulations.

Although the proposals described above exemplify obtaining pharmaceutical forms containing completely soluble Minoxidil when in its primary packaging, the characteristics of the drug in the formulation in contact with the scalp at the time of application and throughout the day must be evaluated. Maintaining the appropriate characteristics of the preparation is essential so that there are no impacts on the availability of the drug.

The pharmacological activity of Minoxidil begins after the conversion of its molecule into Minoxidil sulfate by a sulfotransferase enzyme present in the scalp. Thus, the efficiency and effectiveness of the treatment are directly associated with the concentration of the active molecule reaching the hair bulb of the hair follicle. Furthermore, as it is a product for topical use, it should provide a pleasant feeling to the user, be easy to apply and spread well.

A technical problem seen in Minoxidil-containing formulations is related to the organoleptic properties, mainly the sensorial part. US4828837 reports a composition in which Minoxidil is complexed to an amphipathic compound with a pKa of less than 5, containing a single lipophilic chain and a polar moiety selected from the chemical groups sulfate, sulfonate, phosphate or phosphonates in at least about 1:1. Preferred amphipathic compounds include sulfosuccinic acid hemi-esters, alkyl phosphonates, phosphate esters, and ethoxylated acid hemi-esters, such as the free acids or lauryl ether, lauryl or oleamido polyethylene glycol sulfosuccinate. The document proposes an ointment or cream as the final pharmaceutical form for the product, in an aqueous vehicle and preferably dispersed in fluorochlorocarbon for application in aerosol spray. However, due to the high viscosity of the mixture, the patient will probably feel discomfort when using it, since it is a leave-on product (no-rinse formulation that must be used on the scalp when the hair is clean), with an oily and sticky feeling.

US5030442 shows skin permeation data demonstrating a significant increase in drug permeation only in solutions containing the maximum amount of 2% Minoxidil, showing a limitation of the formulation in supporting greater amounts of drug.

Other examples containing high concentrations of surfactant, above 10% of the formulation in mass/volume, which could increase the drug solubility may pose a great risk to the patient, since Minoxidil must not reach the systemic circulation. If this occurs, drug safety problems may be triggered by overexposure to the drug. In addition, surfactants of sulfated origin have a cleaning power that promotes exacerbated hygiene, contributing to dryness and loss of natural properties of the strand, such as elasticity, resistance and shine.

The prior art reports the crystallization of actives in pharmaceutical preparations containing 5% Minoxidil and, until now, formulations sufficiently capable of avoiding this fact have not been proposed. Additionally, the presence of solid particles can lead to undesirable effects such as irritation, itching, redness, scalp dryness and allergic contact dermatitis, directly impacting patient adherence to treatment. For these reasons, there is a need for new preparations preventing or attenuating the formation of crystals during storage and after application to the scalp, which have topical action on the hair follicles and have pleasant feeling characteristics.

### Brief Description of the Figures

Figure 1 shows: (A) and (B) foam formed by the foamable aqueous solution TEST 4, using a vial with foaming pump supplied by the company Emphasys^{®}; and (C) Optical Transmission Microscopy image of the foam from TEST 4 at 2.5x magnification.
Figure 2 shows: photographs of Petri dishes containing the Rogaine^{®} product foam (1A to 1C) and foam formed by the foamable aqueous solution of the present invention obtained from Example 2 (2A to 2C), respectively, initially (A), after 1 h (B) and after 12 h (C) under ambient conditions (20 °C and 60% RH); Optical Microscopy images with polarized light obtained with 5x magnification indicating the formation of microcrystals after 12 h in the foam of the Rogaine^{®} product (1D) and absence of crystals after 12 h in the foam formed by the foamable aqueous solution of Example 2 (2D).

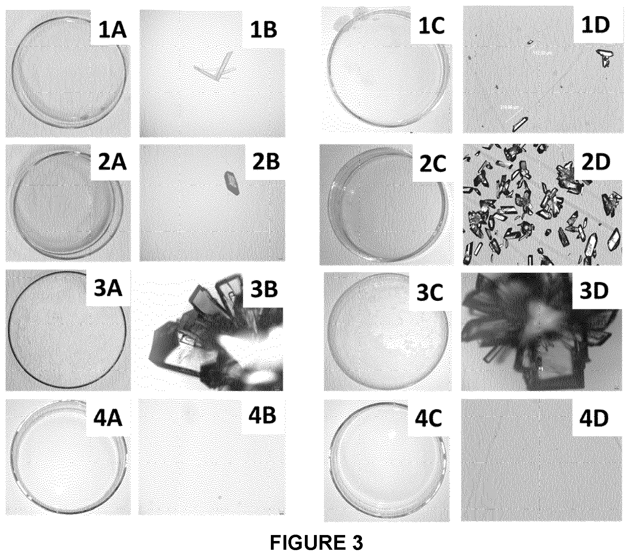

Figure 3 shows: photographs of Petri dishes containing solutions of products Aloxidil^{®} (1A and 1C), Pant^{®} (2A and 2C), Rogaine^{®} emulsion (3A and 3C) and solution of the present invention obtained from Example 2 (4A and 4C) after 12 h (A) and 24 h (C), respectively, under ambient conditions (20 °C and 60% RH); Optical Microscopy images obtained with 5x magnification indicating the formation of crystals in all products Aloxidil^{®} (1B and 1D), Pant^{®} (2B and 2D) and Rogaine^{®} emulsion (3B and 3D) after 12 h (B) and 24 h (D), respectively, except in the solution of Example 2 (4B and 4D).
Figure 4 shows the Raman spectra in the low frequency region for: Minoxidil raw material (A) and after 12 h of exposure at 20 °C and 60% RH for the crystals obtained from commercial products Aloxidil^{®} (B), Pant^{®} (C) and Rogaine^{®} emulsion (D), and for the solution of the present invention obtained from Example 2 (E) and its foam (F) both with visible absence of crystals.

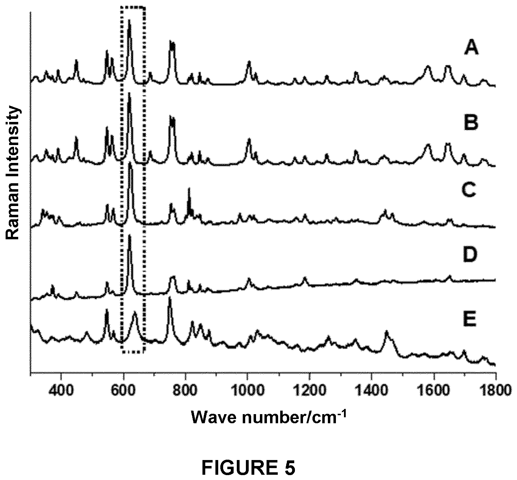

Figure 5 shows the Raman spectra in the mid-frequency region for: (A) Minoxidil raw material, for the crystals obtained from commercial products Rogaine^{®} emulsion (B) and solutions Aloxidil^{®} (C) and Pant^{®} (D) after 24 h of exposure at 20 °C and 60% RH, and for the residue with no crystals for the solution of the present invention obtained from Example 2 (E), after 12 h of exposure at 20 °C and 60% RH. The dotted rectangle indicates a characteristic band of Minoxidil, wider and less intense in the spectrum of the solution of the present invention compared with the spectra of the crystals formed in the products and in the Minoxidil pure raw material.
Figure 6 shows: photographs of Petri dishes containing the solution of the present invention (A) and the foam generated therefrom (B), after 7 days of exposure at 20 °C and 60% RH. The presence of crystals was not observed even after analysis by Optical Microscopy.
Figure 7 shows the evaluation of the degradation products generated from the composition of the present invention obtained from Example 2, containing 5% in (m/v) Lactic Acid and 5% in (m/v) Minoxidil after 1-month stability study at 40 °C and 50 °C.
Figure 8 shows the evaluation of degradation products generated from the composition of the present invention obtained from Example 2, containing equimolar amounts of Lactic Acid and Minoxidil after 1-month stability study at 40 °C and 50 °C.
Figure 9 shows the skin retention of Minoxidil after 24 h of study in the stratum corneum (A) and in the epidermis + dermis (B) of the solution of the present invention batch 397/19 and the commercial product Aloxidil^{®} batch S906.

### Summary of the Invention

The present invention describes a Minoxidil topical foamable pharmaceutical composition that inhibits the formation of drug crystals. Said composition is in the form of a foamable solution, stored in a vial with an applicator selected from a dropper dispenser, foaming pump or spray, and has the advantages of inhibiting the formation of crystals of the drug and have a pleasant tactile feeling, in addition to a local action on the hair follicles.

The present invention has the following objects as inventive concepts.

The present invention has as its first object the topical pharmaceutical composition in foamable solution, composed of Minoxidil, an organic solvent miscible in water, an organic acid, at least one co-solvent, at least one amphoteric surfactant, at least one non-ionic surfactant, at least an antioxidant, and water.

The present invention has as a second object the use of the pharmaceutical composition for the manufacture of a useful drug for the alopecia treatment and other related disorders, as described in the first object and in its embodiments.

### Detailed Description of the Invention

The present invention relates to a topical foamable pharmaceutical composition composed of Minoxidil, a water-miscible organic solvent, an organic acid, at least one co-solvent, at least one amphoteric surfactant, at least one non-ionic surfactant, at least one antioxidant, and water, obtained in an unexpected and surprising way, since there are no prior art compositions with up to 7% of Minoxidil, with the advantages of inhibiting the crystal formation of the drug, having a pleasant tactile feeling, in addition to a topical action on the hair follicles. Comparative studies of in vitro skin permeation performed with the composition of the present invention and a commercial product proved surprisingly the solution of the present invention has a greater retention of Minoxidil in the epidermis + dermis, where the capillary bulb and the hair follicle, action site of the drug Minoxidil, are located.

In aqueous solution Minoxidil formulations, the presence of an organic solvent miscible in water is of fundamental importance for the solubilization of the drug, whose amount varies from 1% to 50% (m/v). In addition, it acts as a preservative, inhibiting the growth of microorganisms and allowing the product to dry quickly after application. Additionally, for the present invention, the amount of organic solvent should not exceed 50% of the mass/volume composition of the formulation in order not to be limiting to the foaming process at the time of product application.

Additionally, the organic acid, in adequate amounts, has the function of increasing the Minoxidil solubility in aqueous formulations. Examples of organic acids are: lactic acid, uric acid, oxalic acid, L-tartaric acid, D-tartaric acid, gallic acid, formic acid, butyric acid, furoic acid, acetic acid, isovaleric acid, picric acid, pyruvic acid, glycolic acid, caproic acid, sorbic acid, propionic acid, caprylic acid, citric acid, butyric acid, benzoic acid, maleic acid, capric acid, salicylic acid, fumaric acid, trans-cinnamic acid, succinic acid, acetylsalicylic acid, malic acid, and myristic.

In addition to acting as an auxiliary solvent for the formulation, the co-solvent acts as a potent humectant, promoting skin hydration and preventing dryness due to the presence of the organic solvent. The optimal amount of cosolvent in aqueous formulations is 1 to 30% (m/v). Examples of co-solvents are benzyl alcohol, glycerin, propylene glycol, polyethylene glycol, butylene glycol, pentylene glycol or mixtures thereof.

In a formulation, antioxidants play the role of preventing or minimizing the degradation of actives and excipients capable of oxidation. Examples of antioxidants are ascorbic acid, cysteine and derivatives, ascorbyl palmitate, hydroxy butyl toluene (BHT), hydroxybutyl anisol, hypophosphorous acid, monothioglycerol, sodium ascorbate, sodium bisulfite, sodium metabisulfite, sodium formaldehyde, propyl gallate, sodium thiosulfate or mixtures thereof, in an amount ranging from 0.1 to 1% (m/v).

The present invention optionally comprises ingredients used in formulations for topical hair treatment, such as antibacterial agents, chelating systems, pH correctors, emulsifying agents, preservatives, vitamins, perfumes, dyes, thickeners, photoprotective agents, and similar.

Foam is a colloidal dispersion, wherein a gas is dispersed in a liquid. In the case of liquid foams, the presence of surfactants is essential for bubbles to be formed and maintained and subsequently give rise to foam. Several factors can affect its stability, such as liquid density, temperature, surface tension, type and concentration of surfactants, bubble size, among others.

Surfactants are organic compounds having a polar ("head") and a nonpolar ("tail") part in its molecule. They are classified as anionic, cationic, non-ionic or amphoteric, according to the substituent present in the polar part.

Cationic surfactants when ionized in water have a positively charged polar part. In general, they are used in preparations that are washed after use (rinse-off), such as shampoos, hair masks and rinse creams. Due to this fact, cationic surfactants were not considered for the development of embodiments of the present invention.

Anionic surfactants have a negatively charged polar part. They have strong detergent power, that is, they are capable of efficiently removing residues from the hair surface, being mostly used in the composition of soaps and cleaning lotions. As a result, anionic surfactants were not used for the embodiments of the present invention.

Non-ionic surfactants, on the other hand, have uncharged radicals. Its water solubility will depend on the substitutions along the chain, and as a result there is a small variety of water-soluble nonionic compounds. In general, during preparations it is necessary to heat the mixture to increase the kinetics of the surfactant solubilization reaction but most of them precipitate again after cooling. For example, this is the case of the commercial product Oleth-20 (cetearyl glucoside), cited in the prior art as a foam stabilizer. In this way, it is not feasible to prepare the solution pharmaceutical form due to the formation of a second phase in the composition. In addition, such surfactants were not effective in inhibiting Minoxidil recrystallization since they are present in the formulation of the commercial product Rogaine^{®}, wherein crystals of this active were found after application on the surface both in the applied foam and in the emulsion present in the packaging. Examples of non-ionic surfactants derived from polyoxyethylene and polyoxypropylene are lauryl polyglucoside, decyl polyglucoside, fatty acid diethanolamide (fatty acid amides), polyoxyethylene-10-dodecyl ether (C12E10), N,N-dimethyldodecylamine-N-oxide (DDAO) and N,N-dimethyloctylamine-N-oxide (DOAO), polysorbates, sorbitan esters, ethoxylated fatty alcohols and glycol and glycerol esters.

Finally, amphoteric surfactants have both anionic and cationic hydrophilic groups in their structure, which give them the ability to form cations or anions depending on the pH of the solution. Thus, at a suitable pH, these surfactants can be compatible with anionic, cationic and non-ionic surfactants. Examples of this group are N-alkyl and C-alkyl betaine and sultaine, amino phosphatidyl alcohol, 3-(dodecyldimethyl ammonium) propane-1-sulfate (SB-12), 4-(dodecyldimethyl ammonium) butyrate (DAB), cocoamidopropyl betaine, alkylimidazoline and sodium cococarboxyamphoglycinate.

Because it is a topical use medication in which hair washing after use is not foreseen, that is, a leave-on type product, for the embodiments of the present invention, two different types of surfactants were selected having low irritability: (i) non-ionic from 1 to 5% (m/v), preferably polysorbate 20 (Tween^{®} 20), non-ionic surfactant due to its greater water solubility when compared to other polysorbates; and (ii) amphoteric from 2 to 10% (m/v), preferably cocoamidopropyl betaine (Crodateric^{®} CAB30) due to its very low irritability and foam stability property. Within the prior art, no documents were found proposing the ideal combination between the surfactants mentioned for a composition in Minoxidil solution.

From the prior art methods used to evaluate crystal formation, the present invention uses Optical Microscopy and Raman Spectroscopy techniques in the low (10 to 200 cm⁻¹) and medium (300 to 1800 cm⁻¹) frequency regions.

In order to determine the in vitro release of active in topical formulations, such as solutions, creams, gels, lotions, transdermal systems, the present invention uses the technique of vertical diffusion cells such as the Franz cell, equipped with a synthetic or animal membrane.

In view of the above, unexpectedly and surprisingly, the present invention discloses adequate proportions of excipients in order to obtain a foamable Minoxidil aqueous solution composition that inhibits the crystal formation, with a pleasant tactile feeling. In this context, the present invention is defined by the following objects and its embodiments.

In a first object, the topical foamable pharmaceutical composition is a solution comprising:
(a) 1 to 7% (mass/volume) Minoxidil or the pharmaceutically acceptable salt thereof;
(b) 1 to 50% (mass/volume) water-miscible organic solvent;
(c) content of an organic acid in an equimolar amount to that of the drug Minoxidil;
(d) 1 to 30% (mass/volume) of at least one co-solvent;
(e) 2 to 10% (mass/volume) of at least one amphoteric surfactant;
(f) 1 to 5% (mass/volume) of at least one nonionic surfactant;
(g) 0.1 to 1% (mass/volume) of at least one antioxidant; and
(h) water.

According to one embodiment of the present invention, the pharmaceutical composition inhibits the drug crystal formation.

According to one embodiment of the present invention, said water-miscible organic solvent is ethanol.

According to one embodiment of the present invention, said organic acid is selected from lactic acid, uric acid, oxalic acid, L-tartaric acid, D-tartaric acid, gallic acid, formic acid, butyric acid, furoic acid, acetic acid, isovaleric acid, picric acid, glycolic acid, caproic acid, sorbic acid, propionic acid, caprylic acid, citric acid, butyric acid, benzoic acid, maleic acid, capric acid, salicylic acid, fumaric acid, trans-cinnamic acid, succinic acid, acetylsalicylic acid, malic acid and myristic acid. In a preferred embodiment, said organic acid is lactic acid.

According to one embodiment of the present invention, said co-solvent is selected from among glycerin, propylene glycol, polyethylene glycol, butylene glycol, pentylene glycol or mixtures thereof. In a preferred embodiment, said co-solvent is glycerin.

According to one embodiment of the present invention, said amphoteric surfactant is selected from N-alkyl and C-alkyl betaine and sultaine, amino phosphatidyl alcohol, 3-(dodecyldimethyl ammonium) propane-1-sulfate (SB-12), 4- (dodecyldimethyl ammonium) butyrate (DAB), cocoamidopropyl betaine, alkylimidazoline, sodium cococarboxyamphoglycinate or mixtures thereof. In a preferred embodiment, said amphoteric surfactant is cocoamidopropyl betaine.

According to one embodiment of the present invention, said non-ionic surfactant is selected from polyoxyethylene and polyoxypropylene derivatives, lauryl polyglucoside, decyl polyglucoside, fatty acid diethanolamide (fatty acid amides), polyoxyethylene-10-dodecyl ether (C12E10), N,N-dimethyldodecylamine-N-oxide (DDAO) and N,N-dimethyloctylamine-N-oxide (DOAO), polysorbates, sorbitan esters, ethoxylated fatty alcohols, glycol and glycerol esters or mixtures thereof. In a preferred embodiment, said nonionic surfactant is polysorbate 20.

According to one embodiment of the present invention, said antioxidant is selected from ascorbic acid, cysteine and derivatives, ascorbyl palmitate, hydroxybutyltoluene (BHT), hydroxybutylanisole, hypophosphorous acid, monothioglycerol, sodium ascorbate, sodium bisulfite, sodium metabisulfite, sodium formaldehyde, propyl gallate, sodium thiosulphate or mixtures thereof. In a preferred embodiment, said antioxidant is hydroxybutyltoluene (BHT).

According to one embodiment of the present invention, the foamable solution comprises:
(a) 1 to 7% (mass/volume) Minoxidil or the pharmaceutically acceptable salt thereof;
(b) 1 to 50% (mass/volume) ethanol;
(c) lactic acid content in an equimolar amount to that of the drug Minoxidil;
(d) 1 to 30% (mass/volume) glycerin;
(e) 2 to 10% (mass/volume) cocoamidopropyl betaine;
(f) 1 to 5% (mass/volume) polysorbate 20;
(g) 0.1 to 1% (mass/volume) hydroxybutyltoluene (BHT); and
(h) water.

According to an embodiment of the present invention, the storage of the pharmaceutical composition, defined in the first object and in its embodiments, is performed in a vial. In a preferred embodiment, said vial contains an applicator selected from a dropper dispenser, foaming pump or spray.

In a second object, the use of the pharmaceutical composition, defined in the first object and in its embodiments, comprises the manufacture of a useful medicine for the treatment of alopecia and other related disorders.

### Examples

The following examples serve to illustrate aspects of the present invention without having, however, any limiting character.

### Example 1 - Feeling test with different surfactants proportions.

Fourteen placebos, that is, minoxidil-free, foamable aqueous solutions were prepared containing different percentages (m/v) of Polysorbate 20 and Cocoamidopropyl Betaine surfactants, as described in Table 1.

Feeling tests were performed on the hands of 5 volunteers, all men, after hand washing using the 14 preparations. For the characterization of the foam, a comparison was made based on the residence time of the foam in the end user's hand and feeling after product application.

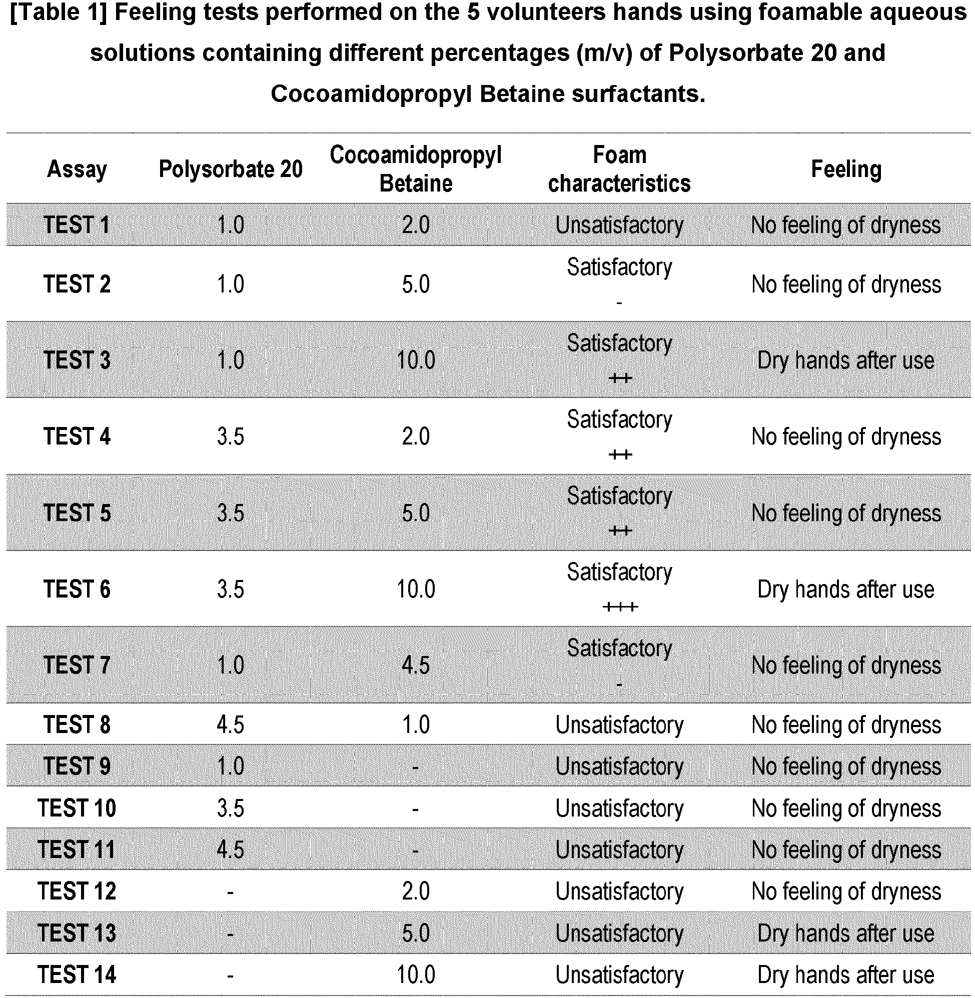

TEST 4 was the solution having a foam closer to the desired characteristics for the product, with a slow drainage of liquid, sufficient residence time to allow the application of the formulation (approximately 2 minutes) and providing a pleasant feeling to the end user. Although surfactants have the ability to form bubbles, as seen experimentally, the use of only one of them is not capable of generating a satisfactory foam.

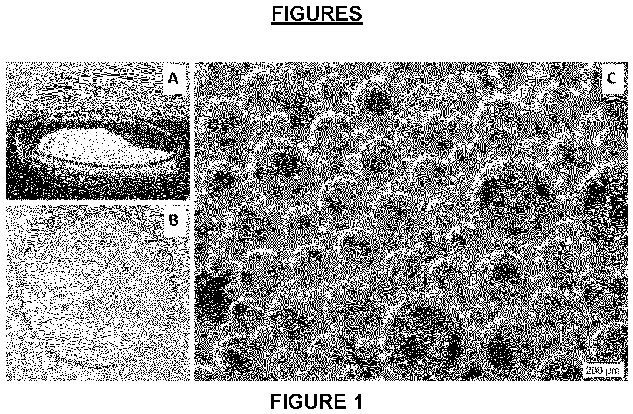

Figure 1 shows the foam formed by the preferred pharmaceutical composition of the invention, according to TEST 4, containing 3.5% (m/v) Polysorbate 20 and 2.0% (m/v) Cocoamidopropyl Betaine, with the use of a foaming pump provided by the company Emphasys^{®} and the analysis by Optical Microscopy of the foam, evidencing the adequate texture and consistency for the application of the product.

Higher amounts of surfactants, in addition to feeling issues, could have an impact during the drug filling process, leading to an excessive variation in the volume of the vials due to the formation of foam during the process.

### Example 2 - Minoxidil foamable aqueous solution composition at 50 mg/mL (5% (m/v)) and its production process.

The ingredients used to obtain a Minoxidil foamable aqueous solution composition 50 mg/mL (5% (m/v)) are described in Table 2.

**[Table 2] Quantitative composition of Minoxidil foamable aqueous solution at 50 mg/mL (5% (m/v)).**

| **Ingredient** | **Quantity (mg/mL)** | **Percentage (m/v)** |
|---|---|---|
| Minoxidil | 50.000 | 5.0 |
| 96% Ethanol | 200.000 | 20.0 |
| Lactic Acid | 30.000 | 3.0 |
| White Glycerin | 120.000 | 12.0 |
| Polysorbate 20 | 35.000 | 3.5 |
| Cocoamidopropyl Betaine | 20.000 | 2.0 |
| BHT | 1.000 | 0.1 |
| Purified water Q.S. | 544.000 | 54.4 |

The production process of the Minoxidil composition was performed in a stainless-steel reactor with a capacity of 500 liters on constant agitation at room temperature and protected from light, which remained closed during the process in order to avoid the evaporation of Ethanol.

Initially, Ethanol and BHT were added in order to guarantee the complete dissolution of the antioxidant at a speed of 50 to 200 rpm. Then, Lactic Acid and Glycerin were added. After 5 minutes, Minoxidil was added to the reactor. After active solubilization, Polysorbate 20, Cocoamidopropyl Betaine and Water were added, with a decrease in agitation speed to 50 to 150 rpm to avoid foam formation. After the end of manipulation, the pH of the formulation was measured and, finally, the product was packaged in suitable packaging.

Alternatively, the Minoxidil foam composition was prepared, initially adding Ethanol, Lactic Acid and Glycerin. After 5 minutes, Minoxidil was added to the reactor at a speed of 50 to 200 rpm. In an auxiliary reactor, Polysorbate 20 and BHT were added. After solubilization of the antioxidant BHT, cocoamidopropyl betaine was added. The mixture was then added to the main reactor and the final volume completed with water, with a decrease in stirring speed to 50 to 150 rpm to avoid foam formation. After the end of manipulation, the formulation pH was measured and finally the product was filled in suitable packaging.

### Example 3 - Comparative study of crystal formation.

A crystallization study was performed in order to demonstrate that the currently commercialized formulations in solution, Pant^{®} and Aloxidil^{®}, and foam, Rogaine^{®}, after application on a surface, have the formation of small crystals, some visible to naked eye and others visible only through Optical Microscopy.

In the package insert of medications containing Minoxidil, patients are warned about the possibility of crystallization in the application valve but there is no mention of the possibility of crystallization in the scalp.

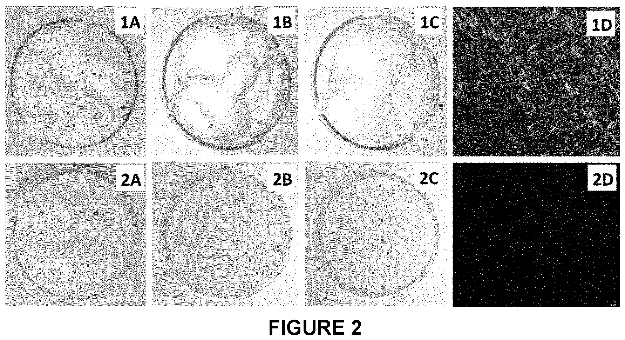

The commercial product in foam form, Rogaine^{®} was applied on a Petri dish and the foam was evaluated right after application, after 1 h and 12 h when kept at 20 °C and 60% relative humidity (RH). The same test was performed for the composition of the present invention, obtained in Example 2 in order to evaluate whether it is capable of inhibiting the formation of Minoxidil crystals. Figure 2 shows photographs of Petri dishes containing the product foam Rogaine^{®} (1A to 1C) and the foam formed by the foamable aqueous solution of the present invention obtained from Example 2 (2A to 2C), respectively, initially, after 1 h and after 12 h exposure under ambient conditions (20 °C and 60% RH); and images of Optical Microscopy with polarized light obtained with 5x magnification, which indicates the formation of microcrystals after 12 h in the foam of the product Rogaine^{®} (1D), showing the presence of microcrystals, and absence of crystals after 12 h in the foam formed by the solution aqueous foam of Example 2 (2D).

In another test, commercial products in Pant^{®} and Aloxidil^{®} solution and Rogaine^{®} emulsion, and the solution of the present invention obtained from Example 2 were applied on Petri dishes, kept at 20 °C and 60% relative humidity and evaluated after 12 h and 24 h. Figure 3 shows photographs of Petri dishes containing solutions of products Aloxidil^{®} (1A and 1C), Pant^{®} (2A and 2C), Rogaine^{®} emulsion (3A and 3C) and solution of the present invention obtained from Example 2 (4A and 4C) after 12 h (A) and 24 h (C), respectively, under ambient conditions (20 °C and 60% RH); Optical Microscopy images obtained with 5x magnification that indicate the formation of crystals in all products Aloxidil^{®} (1B and 1D), Pant^{®} (2B and 2D) and Rogaine^{®} emulsion (3B and 3D) products after 12 h (B) and 24 h (D), respectively, except in the solution of Example 2 (4B and 4D).

In addition to optical microscopy, the crystals formed and the foam residue were evaluated by Raman Scattering Spectroscopy. Raman Microscopy allows obtaining chemical and structural information on materials with micrometric resolution.

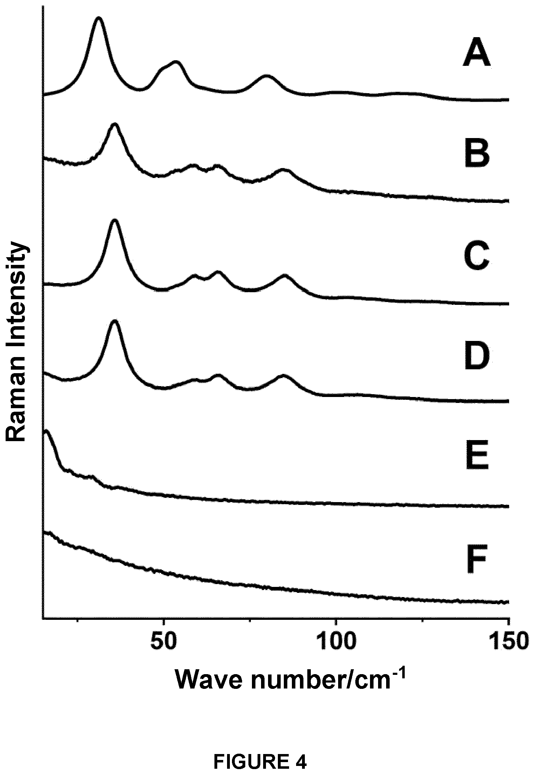

For spectral comparison purposes, the Raman spectrum for the pure drug in crystalline form was obtained. The Raman spectra in the low frequency region (10 to 200 cm⁻¹) and in the medium frequency region (300 to 1800 cm⁻¹) are presented respectively in Figures 4 and 5, for Minoxidil (A) and after 12 h of exposure at 20 °C and 60% RH for the crystals obtained from commercial products in the form of Aloxidil^{®} solution (B), Pant^{®} (C) and in the form of Rogaine^{®} emulsion (D), and for the solution of the present invention obtained from Example 2 (E) and its foam (F), both of them with visible absence of crystals.

The low frequency region containing intermolecular (or crystal lattice) vibration modes in the Raman spectrum can be used to characterize crystalline and amorphous materials. Analysis of the Raman spectra in Figure 4 indicates that the crystals formed in the Pant^{®} (Aché) and Aloxidil^{®} (TheraSkin) solutions and in the Rogaine^{®} emulsion are characteristic of Minoxidil in crystalline form. In the case of an amorphous or semicrystalline material, a spectral profile with broad bands would be observed. This profile is checked for Rogaine^{®} foam that features microcrystals as shown in Figure 2.

The analysis of spectra in the mid-frequency region containing intramolecular vibrational modes can also be used for inferences about the crystallinity of a material. In Figure 5, a thinner bandwidth is seen in the Raman spectra of the drug and products containing Minoxidil crystals.

The Raman spectra of the solution of the present invention indicate a pattern of amorphous material, as can be seen by the greater width of the bands in the Raman spectra shown in Figures 4 and 5, respectively, for the low and medium frequency regions. For example, in Figure 5, the band at approximately 630 cm⁻¹ attributed to Minoxidil is wider and less intense in the spectrum of the solution of the present invention compared to the spectra of pure Minoxidil crystals and formed in commercial products. This result shows the lower tendency of Minoxidil to crystallize in the solution of the present invention in relation to equivalent commercial products containing solutions or foam, even after the evaporation of the volatiles of the formulation.

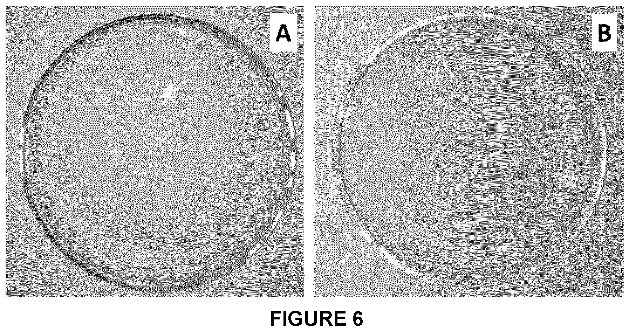

Figure 6 shows the photographs of the Petri dish containing a solution prepared according to the teachings of Example 2 and applied on a Petri dish, kept at 20 °C and 60% RH, for 7 days. The presence of crystals was not observed even after analysis by optical microscopy.

The solution of the present invention and the foam generated therefrom also did not show crystal formation when exposed to a temperature of 40 °C. Even under more severe exposure conditions, for example using a rotary evaporator under reduced pressure at 50 °C, crystal formation was not observed. In this case, the remaining residue of darker coloration have a viscous appearance, however without the presence of crystals when observed under the Optical Microscope.

### Example 4 - Correlation between amount of organic acid and degradation products.

A relevant issue to be highlighted in the formulation is related to the amounts of organic acid, preferably Lactic Acid, and Minoxidil.

US6946120 reports the need to use at least equal or greater amounts by mass of organic acid in relation to the amount of Minoxidil present in the formulation, that is, for a 5% composition, the amount of organic acid must be equal to or greater than 5%.

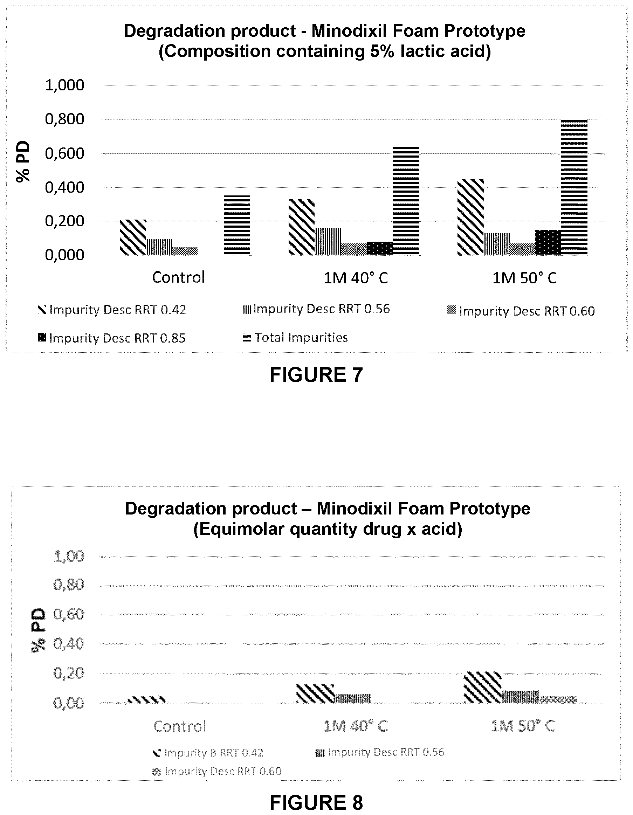

However, experimentally, it was observed that for the composition of the present invention prepared with the same amount of 5% in (m/v) of Lactic Acid and Minoxidil, there is a greater drug degradation (see Table 3 and Figure 7) when compared to the composition with Minoxidil in equimolar amounts with Lactic Acid (see Table 4). In addition, excess acid favors the darkening of the product, providing it with inadequate visual characteristics.

**[Table 3] Analysis of degradation products generated from the composition of the present invention obtained from Example 2, containing 5% in (m/v) of Lactic Acid and 5% in (m/v) of Minoxidil, after 1-month stability study at 40 °C and 50 °C.**

| Incubation Condition | Impurity Desc RRT 0.42 | Impurity Desc RRT 0.56 | Impurity Desc RRT 0.60 | Impurity Desc RRT 0.85 | Total Impurities |
|---|---|---|---|---|---|
| Control | 0.210 | 0.096 | 0.046 | - | 0.352 |
| Incubator: 1 month at 40° C±75% RH | 0.330 | 0.160 | 0.07 | 0.080 | 0.640 |
| Incubator: 1 month at 50° C±75% RH | 0.450 | 0.130 | 0.070 | 0.150 | 0.800 |

**[Table 4] Analysis of degradation products generated from the composition of the present invention obtained from Example 2 containing equimolar amounts of Lactic Acid and Minoxidil, considering a 5% Minoxidil solution, after 1-month stability study at 40 °C and 50 °C.**

| Incubation Condition | Impurity Desc RRT 0.42 | Impurity Desc RRT 0.56 | Impurity Desc RRT 0.60 | Impurity Desc RRT 0.85 | Total Impurities |
|---|---|---|---|---|---|
| Control | 0.05 | 0.000 | 0.000 | - | 0.050 |
| Incubator: 1 month at 40° C±75% RH | 0.13 | 0.059 | 0.000 | - | 0.189 |
| Incubator: 1 month at 50° C±75% RH | 0.21 | 0.081 | 0.046 | 0.080 | 0.417 |

Thus, for the present invention, the amounts of organic acid and Minoxidil must be equimolar, that is, the amount of acid must be equivalent in number of moles of the drug in order to increase the stability of the formulation.

A similar fact was noticed in tests considering other acids such as acetic acid and pyruvic acid.

### Example 5 - In vitro skin permeation study

To determine the in vitro release of active in topical formulations, such as solutions, creams, gels, lotions, transdermal systems, among others, regulatory agencies and scientific literature recommend the use of vertical diffusion cells, such as the Franz cell, equipped with synthetic or animal membrane.

The Franz cell follows the bicompartmental model of diffusion cells, having two compartments, one containing the drug (donor compartment) and the other containing a solution where the drug is soluble, separated by a natural membrane.

Measuring the extent to which a formulation yields is fundamental for evaluating a dermatological formulation. After release of the formulation, penetration through the stratum corneum will determine the local availability of the drug. Determining the drug amount reaching the action site is another important measure that can predict the effectiveness of the dermatological product. The amount needed to trigger the pharmacological effect will depend on the intrinsic efficacy of the drug.

The results of the skin retention tests showed that there is retention of 36.74 µg·cm⁻² and 30.66 µg·cm⁻² of Minoxidil in the stratum corneum, after 24 h of study, for the solution of the present invention of Minoxidil at 50 mg/mL Batch: 397/19 (Test) obtained from Example 2 and the commercial product Aloxidil^{®} Batch: S906 (comparator), respectively. Statistical comparison showed that there is no significant difference for Minoxidil retention in the stratum corneum between the samples, as shown in Table 5.

**[Table 5] Skin retention of Minoxidil, in the stratum corneum, after a 24 h study comparing the solution of the present invention obtained from Example 2 (Batch 397/19 (Test)) versus the Aloxidil^{®} solution Batch: S906.**

| **Copy** | **Minoxidil 50 mg/mL, B: 397/19 (Test)** | | **Aloxidil^{®} B: S906 (Comparator)** | |
|---|---|---|---|---|
| | **µg·cm-2** | **% active** | **µg·cm-2** | % active |
| 1 | 19.56 | 0.23 | 40.31 | 0.48 |
| 2 | 43.72 | 0.52 | 21.73 | 0.26 |
| 3 | 40.61 | 0.48 | 30.62 | 0.36 |
| 4 | 23.98 | 0.28 | 38.91 | 0.46 |
| 5 | 44.78 | 0.53 | 24.15 | 0.28 |
| 6 | 47.77 | 0.56 | 28.25 | 0.33 |
| Average | 36.74 | 0.43 | 30.66 | 0.36 |
| Standard deviation | 11.90 | 0.14 | 7.60 | 0.09 |
| DPR (%) | 32.40 | 32.40 | 24.80 | 24.80 |

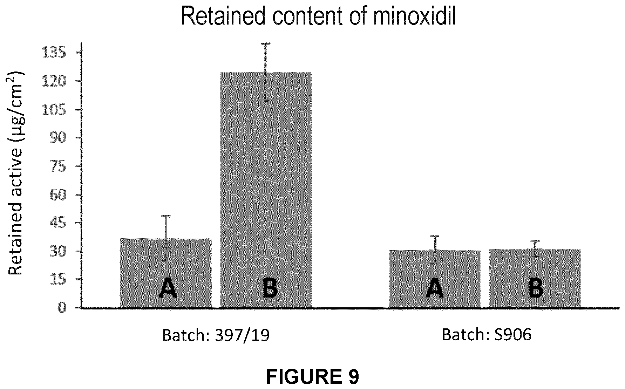

The result also showed that 124.70 µg·cm⁻² and 31.21 µg·cm⁻² of Minoxidil are retained in the epidermis + dermis, after 24 h of study, for the Test products at 50 mg/mL Batch: 397/19 and Aloxidil^{®} Batch: S906 (comparator), respectively. This retention represents respectively 1.47% and 0.37% of the total Minoxidil contained in the formulations. Statistical comparison showed that there is a significant difference for Minoxidil retention in the epidermis + dermis between the samples. The results of the in vitro study of the Test sample indicate a greater availability of the drug in the dermis layer, where the hair bulb and the hair follicle are located, site of action of Minoxidil, as shown in Table 6 and Figure 9.

**[Table 6] Skin retention of Minoxidil, in the epidermis + dermis, after a 24-hour study comparing Test solutions (Batch 397/19) versus Aloxidil^{®} Batch: S906.**

| **Copy** | **Minoxidil 50 mg/mL, B: 397/19 (Test)** | | **Aloxidil^{®} B: S906 (Comparator)** | |
|---|---|---|---|---|
| | **µg·cm⁻²** | **% active** | **µg·cm⁻²** | **% active** |
| 1 | 154.13 | 1.82 | 29.40 | 0.35 |
| 2 | 128.20 | 1.51 | 24.78 | 0.29 |
| 3 | 115.44 | 1.36 | 32.02 | 0.38 |
| 4 | 117.64 | 1.39 | 30.68 | 0.36 |
| 5 | 116.35 | 1.37 | 37.15 | 0.44 |
| 6 | 116.44 | 1.37 | 33.20 | 0.39 |
| Average | 124.70 | 1.47 | 31.21 | 0.37 |
| Standard deviation | 15.18 | 0.18 | 4.12 | 0.05 |
| DPR (%) | 12.17 | 12.17 | 13.20 | 13.20 |

It should be understood that the embodiments described above are merely illustrative and that any modifications thereto may occur to a person skilled in the art. Consequently, the present invention should not be considered limited to the embodiments described in the present specification.

## Claims

1. A topical foamable pharmaceutical composition provided as a solution comprising:
(a) 1 to 7% (mass/volume) Minoxidil or the pharmaceutically acceptable salt thereof;
(b) 1 to 50% (mass/volume) water-miscible organic solvent;
(c) content of an organic acid in an equimolar amount to that of the drug Minoxidil;
(d) 1 to 30% (mass/volume) of at least one co-solvent;
(e) 2 to 10% (mass/volume) of at least one amphoteric surfactant;
(f) 1 to 5% (mass/volume) of at least one nonionic surfactant;
(g) 0.1 to 1% (mass/volume) of at least one antioxidant; and
(h) water.

2. The pharmaceutical composition according to claim 1, ***characterized in that*** said organic solvent is ethanol.

3. The pharmaceutical composition according to claim 1 or 2, ***characterized in that*** said organic acid is selected from lactic acid, uric acid, oxalic acid, L-tartaric acid, D-tartaric acid, gallic acid, formic acid, butyric acid, furoic acid, acetic acid, isovaleric acid, picric acid, glycolic acid, caproic acid, sorbic acid, propionic acid, caprylic acid, citric acid, butyric acid, benzoic acid, maleic acid, capric acid, salicylic acid, fumaric acid, trans-cinnamic acid, succinic acid, acetylsalicylic acid, malic acid and myristic acid.

4. The pharmaceutical composition according to any one of claims 1 to 3, ***characterized in that*** said co-solvent is selected from glycerin, propylene glycol, polyethylene glycol, butylene glycol, pentylene glycol or mixtures thereof.

5. The pharmaceutical composition according to any one of claims 1 to 4, ***characterized in that*** said amphoteric surfactant is selected from N-alkyl and C-alkyl betaine and sultaine, phosphatidyl amino alcohol, 3-(dodecyldimethyl ammonium) propane-1-sulfate (SB-12), 4-(dodecyldimethyl ammonium) butyrate (DAB), cocoamidopropyl betaine, alkylimidazoline, sodium cococarboxyamphoglycinate or mixtures thereof.

6. The pharmaceutical composition according to any of the preceding claims, ***characterized in that*** said nonionic surfactant is selected from derivatives of polyoxyethylene and polyoxypropylene, lauryl polyglucoside, decyl polyglucoside, fatty acid diethanolamide (fatty acid amides), polyoxyethylene-10-dodecyl ether (C12E10), N,N-dimethyldodecylamine-N-oxide (DDAO) and N,N-dimethyloctylamine-N-oxide (DOAO), polysorbates, sorbitan esters, ethoxylated fatty alcohols, glycol and glycerol esters or mixtures thereof.

7. The pharmaceutical composition according to any of the preceding claims, ***characterized in that*** said non-ionic surfactant is polysorbate 20.

8. The pharmaceutical composition according to any of the preceding claims, ***characterized in that*** said antioxidant is selected from ascorbic acid, cysteine and derivatives, ascorbyl palmitate, hydroxybutyl toluene (BHT), hydroxybutyl anisol, hypophosphorous acid, monothioglycerol, sodium ascorbate, sodium bisulfite, sodium metabisulfite, sodium formaldehyde, propyl gallate, sodium thiosulfate or mixtures thereof.

9. The pharmaceutical composition as defined in any of the preceding claims, ***characterized in that*** it comprises:
(a) 1 to 7% (mass/volume) Minoxidil or the pharmaceutically acceptable salt thereof;
(b) 1 to 50% (mass/volume) ethanol;
(c) lactic acid content in an equimolar amount to that of the drug Minoxidil;
(d) 1 to 30% (mass/volume) glycerin;
(e) 2 to 10% (mass/volume) cocoamidopropyl betaine;
(f) 1 to 5% (mass/volume) polysorbate 20;
(g) 0.1 to 1% (mass/volume) hydroxybutyltoluene (BHT); and
(h) water.

10. The pharmaceutical composition according to any of the preceding claims, ***characterized in that*** it is stored in a vial.

11. The pharmaceutical composition according to claim 10, wherein said vial further contains an applicator selected from a dropper dispenser, foaming pump or spray.

12. The pharmaceutical composition as defined in any one of claims 1 to 11, for use in the treatment of alopecia and/or other related disorders.

13. Use of the pharmaceutical composition according to any of claims 1 to 11 for the inhibition of the formation of active Minoxidil crystals.

14. The use according to claim 13, ***characterized in that*** the formation of Minoxidil crystals is inhibited at a temperature of up to at least 40 °C.
